# EUROPEAN PATENT SPECIFICATION

(11) **EP 0 449 787 B1**
(45) Date of publication and mention of the grant of the patent: **27.09.1995**
(21) Application number: 91830107.8
(22) Date of filing: 19.03.1991
(51) Int. Cl.: A61K 38/05

(54) **Pharmaceutical, dietetic or veterinary compositions with eumetabolic activity**
Pharmazeutische, diätetische oder veterinäre Zusammensetzungen mit eumetabolischer Aktivität
Compositions pharmaceutiques, diététiques ou vétérinaires à activité eumétabolique

(30) Priority: 21.03.1990 IT 1975490
(43) Date of publication of application: 02.10.1991
(73) Proprietor: SETRA S.r.l., I-20099 Sesto S.Giovanni (Milan) (IT)
(72) Inventor: Negrisoli, Gian Paolo, I-24100 Bergamo (IT)
(74) Representative: Minoja, Fabrizio

(56) References cited:
- WO-A-90/06102
- US-A- 4 446 149

## Description

The present invention relates to the use of carnosine or peptides related thereto for the preparation of medicaments for the treatment of muscular fatigue and improving athletic performances in persons subjected to prolonged physical efforts.

Carnosine is a physiological substance, which is present in muscles and in some nervous tissues of mammals and, particularly, of man, in concentrations ranging from 15 to 40 mmoles/ kg of tissue. Chemically, carnosine consists of a dipeptide, namely β-alanyl-L-histidine.

The physiological action of this peptide is well-known and it is expressed at intracellular level by buffering the exceeding protons, which come from the physiological processes involved in energy production.

In fact, after an increased muscular or cerebral activity, the so called "breathing-acidosis" occurs and, after an even stronger activity, glycogen degradation gives rise to lactic acid accumulation in tissues, and the consequent "metabolic acidosis" occurs.

In both cases uncontrolled proton release would cause an intracellular pH drop, so as to compromise the phosphocreatine-creatine phosphokinase system, which is involved in the regeneration of ADP to ATP; the failure of said system is probably one of the main causes of muscular fatigue.

Intracellular buffering agents act by shifting the point where phosphocreatine-creatine phosphokinase system blockage occurs, therefore lowering weakening sensation.

All dipeptides with pKa near physiological pH can act as intracellular buffering agents: in addition to carnosine, other dipeptides containing histidine imidazole ring can be used such as :
homocarnosine: α-aminobutyryl-L-histidine
anserine: β-alanyl-L-1-methyl-histidine
homoanserine:α-aminobutyryl-L-1-methyl-histidine
ophidine: β-alanyl-L-3-methyl-histidine.

US-A-4,446,149 discloses pharmaceutical compositions comprising L-carnosine for the treatment of cervical erosion.

WO-A-90 06102 discloses compositions for reducing or preventing collagen cross-linking in skin and/or damage to skin cell DNA comprising, inter alia, carnosine or analogs thereof.

It has now been found that administering these substances in the form of pharmaceutical, dietetic or veterinary compositions to human or animal organisms is capable of causing beneficial effects, which are unpredictable from what is known about the biochemistry and the metabolism of said physiological substances.

Examples of such beneficial effects include athletic performance improvement in persons subjected to prolonged efforts, improvement of muscular functional capacity in elderly or weakened subjects, in children, in dialysed patients or in those patients with a reduced hepatic functional capacity. Carnosine administration proved also to be advantageous in breeding, particularly in improving horse performances in equestrian sports. Therefore, according to the invention oral pharmaceutical , dietetic or veterinary compositions containing a dipeptide selected from the group of carnosine, homocarnosine, anserine, homoanserine or ophidine or physiologically equivalent derivatives thereof such as salts, acetyl derivatives and the like are provided. Said compositions can further contain other active ingredients with complementary or anyway useful activities such as creatine, carnitine, acetylcarnitine, essential or non essential amino acids, sugars, mineral salts and vitamins. Carnosine is the preferred dipeptide, whose daily posology can vary within broad limits, because of its extremely low toxicity. In therapeutic or dietetic use, daily posology will generally range between 300 mg and 5 g a day. Other optional active ingredients, which are selected depending on the specific indications foreseen for the compositions of the invention, will be present in such an amount to provide a suitable supply of said substances.

For instance creatine can be administered in doses from 0.5 to 10 g a day; carnitine in doses from 100 mg to 2 g a day, amino acids from 100 mg to 5 g a day; always referring to human therapeutic or dietetic use.

The compositions of the invention are prepared using conventional techniques and excipients, like the ones described in "Remington's Pharmaceutical Sciences Handbook", Mack Pub. Co., N.Y., U.S.A.

Examples of formulations include single-dose sachets containing powders or granulates which can optionally be effervescent and can be dissolved in water or other beverages before use; tablets; soft or hard capsules; syrups; sweets and the like; containing not only carnosine and optionally other active ingredients, but also appropriate excipients such as flavouring, sweetening, effervescence agents and all those additives which are well-known to the man skilled in the art.

The following examples further illustrate the invention.

### EXAMPLE 1

Composition, to be orally administered from four times to twice a day as support to a strong muscular activity, containing carnosine, essential amino acids and sugars, in the following proportions:

| | |
|---|---|
| Carnosine | 1.0 g |
| Histidine | 1.0 g |
| Carnitine | 0.5 g |
| Creatine | 2.0 g |
| Glucose | 1.0 g |
| Flavouring agents | |

### EXAMPLE 2

Composition, to be administered to subjects with nutritional or liver metabolic deficiency or in case of muscular fatigue from three times to once a day, containing carnosine and essential amino acids in the following proportions:

| | |
|---|---|
| Carnosine | 0.35 g |
| Creatine | 0.75 g |
| Histidine | 0.35 g |
| Carnitine | 0.175 g |
| Flavouring agents | |

### EXAMPLE 3

| | |
|---|---|
| Carnosine | 1.0 g |
| Potassium aspartate | 1.0 g |
| Fructose or dextrose | 1.0 g |
| Sodium hydrogencarbonate | 1.5 g |

### EXAMPLE 4

| | |
|---|---|
| Acetylcarnosine | 0.8 g |
| Histidine | 0.8 g |
| Carnitine | 0.3 g |
| Potassium aspartate | 1.0 g |

## Claims

1. The use of the dipeptides selected from the group of carnosine, homocarnosine, anserine, homoanserine, ophidine or physiologically equivalents thereof for the preparation of medicaments for treating muscular fatigue states and for improving athletic performances in persons subjected to prolonged physical efforts.

## Patentansprüche

1. Die Verwendung von Dipeptiden, ausgewählt unter der Gruppe von Carnosin, Homocarnosin, Anserin, Homoanserin, Ophydin oder ihren physiologischen Äquivalenten für die Vorbereitung von Heilmitteln für die Behandlung von Muskelmüdigkeitszuständen und für die Verbesserung von athletischen Leistungen bei Personen, die sich langen physischen Anstrengungen unterwerfen.

## Revendications

1. L'emploi de dipeptides choisis dans le groupe de carnosine, homocarnosine, anserine, homoanserine, ophidine ou leurs équivalents physiologiques pour la préparation de médicaments pour le traitement des états de lassitude musculaire et pour l'amelioration des performances athlétiques chez personnes soumises à efforts physiques prolongés.
